# EUROPÄISCHE PATENTSCHRIFT

(11) **EP 3 246 067 B1**
(45) Veröffentlichungstag und Bekanntmachung des Hinweises auf die Patenterteilung: **26.09.2018**
(21) Anmeldenummer: 17000842.9
(22) Anmeldetag: 17.05.2017
(51) Int. Cl.: A61M 37/00

(54) **NADELMODUL FÜR EIN HAUTPUNKTIERUNGSGERÄT UND HAUTPUNKTIERUNGSGERÄT MIT DERARTIGEM NADELMODUL**
NEEDLE MODULE FOR A SKIN PUNCTURING APPARATUS AND SKIN PUNCTURING APPARATUS COMPRISING SUCH A NEEDLE MODULE
MODULE D'AIGUILLE POUR UN APPAREIL DE PERFORATION DE PEAU ET APPAREIL DE PERFORATION DE PEAU COMPRENANT UN TEL MODULE D'AIGUILLE

(30) Priorität: 18.05.2016 DE 102016006186
(43) Veröffentlichungstag der Anmeldung: 22.11.2017
(73) Patentinhaber: Dermaroller GmbH, 38304 Wolfenbüttel (DE)
(72) Erfinder: Tomerius, Michael, 38304 Wolfenbüttel (DE)
(74) Vertreter: Tomerius, Isabel

(56) Entgegenhaltungen:
- EP-A1- 2 954 926
- WO-A1-2011/048482
- CN-Y- 2 275 851
- DE-U1-202010 013 095
- US-A1- 2012 192 681
- US-A1- 2016 038 176
- US-A1- 2016 074 646

## Beschreibung

Die Erfindung betrifft ein Nadelmodul zum Anschluss an ein Antriebsmodul eines Hautpunktierungsgerätes. Darüber hinaus betrifft die Erfindung ein Hautpunktierungsgerät mit einem derartigen Nadelmodul.

Gattungsgemäße Hautpunktierungsgeräte werden zur medizinischen und/oder kosmetischen Behandlung der menschlichen und/oder tierischen Haut eingesetzt. Es handelt sich dabei um Handgeräte, die von einer behandelnden Person präzise über die Haut der behandelten Person geführt werden. Beispielsweise ist es bekannt, durch wiederholtes Einstechen in die Haut mittels feinster Nadeln Mikroläsionen zu setzen, die die Wundheilung fördern, beispielsweise bei Verbrennungswunden. Gleichzeitig wird eine Narbenbildung an den Wunden verringert. Darüber hinaus können Hautpunktierungsgeräte ebenfalls eingesetzt werden, um Wirkstoffe, beispielsweise Arzneimittel oder Kosmetika, oder auch Farbstoffe oder Pigmente, in die Haut einzubringen. So werden beispielsweise zur Anbringung von Permanent-Make-Up oder Tätowierungen Hautpunktierungsgeräte eingesetzt. Derartige Hautpunktierungsgeräte sind beispielsweise aus der DE 20 2010 013 095 U1, der EP 2 954 926 A1 oder der CN 2 275 851 Y bekannt.

Die gattungsgemäßen Hautpunktierungsgeräte zeichnen sich dadurch aus, dass sie eine oder mehrere Nadeln über einen Antrieb in sich wiederholender Weise hin und her bewegen, so dass diese Bewegung der Nadel bzw. der Nadeln dazu benutzt werden kann, wiederholt in Haut einzustechen. Bei der oder den Nadeln handelt es sich zum Beispiel um besonders feine Mikronadeln, die einen maximalen Durchmesser von 0,1 bis 1 mm, typischerweise von unter 0,25 mm, aufweisen. Hautpunktierungen mittels derartiger Mikronadeln werden durch Zellregeneration narbenlos geschlossen, sodass durch die Behandlung in der Regel keine oder allenfalls minimale und praktisch nicht sichtbare Narbenbildung hervorgerufen wird. Zum Einbringen von Substanzen in die Haut können alternativ auch Kanülen oder Hohlnadeln verwendet werden.

Gattungsgemäße Hautpunktierungsgeräte umfassen ein Nadelmodul und ein Antriebsmodul, wobei das Nadelmodul zum Anschluss an das Antriebsmodul ausgebildet ist. Dazu umfasst das Nadelmodul ein am Antriebsmodul befestigbares Anschlussstück, das beispielsweise auch einstückig mit dem Antriebsmodul bzw. einem Teil des Antriebsmoduls, zum Beispiel einem Gehäuse, ausgebildet sein kann. Das Anschlussstück ist dasjenige Teil des Hautpunktierungsgerätes, über das das Nadelmodul am Antriebsmodul befestigt ist. Darüber hinaus umfasst das Nadelmodul typischerweise eine Triebstange und einen an der Triebstange befestigten Nadelhalter mit wenigstens einer Nadel, wobei die Triebstange derart mit einem Antrieb des Antriebsmoduls verbindbar ausgebildet ist, dass sie eine vom Antrieb kommende Auf- und Abbewegung auf den Nadelhalter überträgt. Üblicherweise umfassen die Nadelmodule weiter eine den Nadelhalter und einen Teil der Triebstange aufnehmende Glocke, die derart ausgebildet ist, dass die wenigstens eine Nadel des Nadelhalters an einer geöffneten Seite durch die Auf- und Abbewegung in Punktionsrichtung aus einem Innenraum der Glocke herausgeschoben und wieder in diesen eingezogen werden kann. Die Glocke bildet also ein Gehäuse, in dem der Nadelhalter derart gelagert ist, dass er gegenüber der Glocke bewegt werden kann, wodurch die Nadel bzw. die Nadeln des Nadelhalters an einer Seite der Glocke aus dieser austreten können. Wird das Hautpunktierungsgerät mit dieser Seite der Glocke auf die Haut aufgelegt, so wird durch die sich wiederholende Auf- und Abbewegung der Nadeln ein wiederholtes Einstechen der Haut erreicht. Die Auf- und Abbewegung geht dabei so weit, dass die wenigstens eine Nadel des Nadelhalters komplett in die Glocke zurückgezogen wird, um sie ebenfalls komplett aus der Haut herauszuziehen. Diejenige Richtung, in der die wenigstens eine Nadel auf und ab bewegt wird, wird als Punktionsrichtung bezeichnet. Insbesondere wird die wenigstens eine Nadel in Punktionsrichtung aus der Glocke herausgeführt und sticht in Punktionsrichtung in die Haut ein, während die wenigstens eine Nadel entgegen der Punktionsrichtung zurück in die Glocke bewegt wird. Weiter umfasst das Nadelmodul eine die Triebstange und die Glocke umschließende, am Anschlussstück befestigte Manschette. Die Manschette befestigt die Glocke am Anschlussstück und damit am Antriebsmodul des Hautpunktierungsgerätes. Dabei ist die Triebstange mit dem Nadelhalter in Punktionsrichtung gleitend in der Glocke und in der Manschette gelagert. Auf diese Weise kann die Triebstange und der Nadelhalter mit der wenigstens einen Nadel relativ zur Glocke, der Manschette und dem Anschlussstück bewegt werden.

Im Betrieb eines derartigen Hautpunktierungsgerätes muss die wenigstens eine Nadel öfter gewechselt werden. Dies kann beispielsweise sein, weil für eine geplante Anwendung eine andere Größe der wenigstens einen Nadel notwendig ist oder weil die derzeit am Hautpunktierungsgerät befindlichen Nadeln bereits benutzt worden sind und aus hygienischen Gründen ausgetauscht werden müssen. Da die medizinische und kosmetische Anwendung der gattungsgemäßen Hautpunktierungsgeräte erfordert, dass die Einstiche in die Haut besonders präzise ausgeführt werden, sind im Stand der Technik teilweise sehr komplexe Kupplungssysteme bekannt, über die die wenigstens eine Nadel bzw. der Nadelhalter eines Nadelmoduls ausgetauscht werden können. Dieser Austausch erfordert dabei teilweise viel Zeit und/oder den Einsatz von Spezialwerkzeugen. Dies führt zu einem höheren Wartungsaufwand und damit höheren Betriebskosten der gattungsgemäßen Hautpunktierungsgeräte.

Vor diesem Hintergrund ist es die Aufgabe der vorliegenden Erfindung, ein Hautpunktierungsgerät bzw. ein Nadelmodul anzugeben, dessen Wartungsaufwand trotz geringer Herstellungskosten verringert ist und das somit geringere Betriebskosten aufweist. Gleichzeitig soll eine präzise Auf- und Abbewegung und eine exakte Führung der wenigstens einen Nadel für größtmögliche Präzision des Hautpunktierungsgerätes gewährleistet sein.

Die Lösung der Aufgabe gelingt mit einem Nadelmodul und einem Hautpunktierungsgerät gemäß den unabhängigen Ansprüchen. Bevorzugte Weiterbildungen sind in den abhängigen Ansprüchen angegeben.

Konkret gelingt die Lösung bei einem eingangs genannten gattungsgemäßen Nadelmodul dadurch, dass die Manschette an einer Seite quer zur Punktionsrichtung offenbar ist, so dass die Triebstange und die Glocke aus der Manschette entfernbar sind. Die erfindungsgemäße Manschette ist zwischen einer die Triebstange und die Glocke umschließenden Position und einer die Triebstange und die Glocke freigebenden Position verstellbar. Erfindungsgemäß wird "umschließen" synonym zu "umgreifen" verwendet. Es reicht also aus, dass die Manschette einen Teil der Glocke umgreift/umschließt. Es muss nicht die gesamte Glocke in der Manschette eingeschlossen sein. In der die Triebstange und die Glocke umschließenden Position der Manschette befestigt die Manschette die Triebstange und die Glocke am Nadelmodul und über das Anschlussstück auch am Antriebsmodul. Insbesondere ist die Glocke in dieser Position unmittelbar, insbesondere ortsfest, bevorzugt formschlüssig, an der Manschette gelagert. In der die Triebstange und die Glocke freigebenden Position dagegen sind die Triebstange und die Glocke nicht am Nadelmodul befestigt und können von diesem entfernt werden. Die Triebstange und die Glocke sind also durch die Öffnung der Manschette quer zur Punktionsrichtung aus der Manschette entfernbar ausgebildet. Nachdem die Manschette quer zur Punktionsrichtung geöffnet wurde, können die Triebstange und die Glocke also durch eine zweckmäßig ebenfalls quer zur Punktionsrichtung ausgeführte Bewegung von der Manschette demontiert werden. Es können dann entweder die Triebstange, die Glocke oder der Nadelhalter insgesamt ausgetauscht werden. Dies kann beispielsweise notwendig sein, wenn ein anders geformter Nadelhalter, der ebenfalls eine anders geformte Glocke benötigt, zum Einsatz kommen soll. Alternativ ist es ebenfalls möglich, die Triebstange aus der Glocke zu ziehen und den Nadelhalter von der Triebstange zu lösen. Auf diese Weise kann lediglich der Nadelhalter mit der wenigstens einen Nadel ausgewechselt werden, wobei ein neuer Nadelhalter wieder an derselben Triebstange befestigt wird, die dann lediglich durch die Glocke hindurch gesteckt werden muss. Danach werden Triebstange und die Glocke in die geöffnete Manschette eingelegt, und die Manschette wird geschlossen. Das Einlegen der Triebstange und der Glocke erfolgt zweckmäßig ebenfalls quer zur Punktionsrichtung. Nachdem die Manschette geschlossen wurde, ist das Hautpunktierungsgerät mit der neuen wenigstens einen Nadel einsatzbereit. Durch die erfindungsgemäß quer zur Punktionsrichtung offenbare Manschette lässt sich der Nadelhalter mit der wenigstens einen Nadel also schnell, unkompliziert und ohne den Einsatz von Spezialwerkzeug auswechseln. Insgesamt wird der Betrieb des Hautpunktierungsgerätes damit wesentlich vereinfacht.

Die Einstichtiefe der Nadel in die Haut beträgt beispielsweise bis zu 0,6 mm und liegt bevorzugt in einem Bereich zwischen 0,1 und 0,6 mm. Je nach Anwendungsgebiet kann eine unterschiedliche Eindringtiefe der Nadeln in die Haut notwendig sein. Es ist daher bevorzugt, das Nadelmodul derart auszubilden, dass die Einstichtiefe nach Bedarf einstellbar ist, insbesondere im beschriebenen Bereich. Eine Verstellung der Einstichtiefe wird dadurch erreicht, dass die Glocke oder ein mit der Glocke verbundener Bereich des Nadelmoduls, der nicht vom Antrieb auf und ab bewegt wird, derart in der Länge verstellbar ausgebildet ist, dass sich die Lage des Öffnungsrandes der Glocke gegenüber der wenigstens einen in der Punktionsrichtung vollständig ausgefahrenen Nadel verändert. Durch diese Lageänderung wird die wenigstens eine Nadel dann bei unveränderter Auf- und Abbewegung des Antriebs entweder weiter oder weniger weit aus der Glocke heraus geschoben, je nachdem, ob der feststehende Bereich des Nadelmoduls verlängert oder verkürzt wird. Wird beispielsweise der feststehende Bereich des Nadelmoduls verlängert, so liegt der Nadelhalter aufgrund seiner konstanten Länge und der konstanten Länge der Triebstange weiter im Innenraum der Glocke als zuvor. Dadurch tritt die wenigstens eine Nadel durch die Auf- und Abbewegung weniger weit durch die geöffnete Seite der Glocke nach außen hervor und kann so weniger tief in die Haut eindringen. Andererseits steht durch eine Verkürzung des feststehenden Bereichs des Nadelmoduls die wenigstens eine Nadel in der maximal ausgeschobenen Position weiter über die Glocke vor, sodass die wenigstens eine Nadel tiefer in die Haut eindringt. Grundsätzlich können verschiedene Bereiche des Nadelmoduls für die Verstellbarkeit der Einstichtiefe genutzt werden. So ist beispielsweise möglich, dass die Glocke und/oder die Manschette und/oder das Anschlussstück eine Einstichtiefenverstelleinrichtung aufweist, die insbesondere als Gewinde ausgebildet ist, wobei die Länge des jeweiligen Bereichs des Nadelmoduls in Punktionsrichtung über die Einstichtiefenverstelleinrichtung verstellbar ist. Bevorzugt weist das Anschlussstück die entsprechende Einstichtiefenverstelleinrichtung auf und ist in seiner Länge in Punktionsrichtung verstellbar. Besonders bevorzugt ist die Einstichtiefenverstelleinrichtung so ausgebildet, dass durch eine Verstellung der Einstichtiefe weder die Manschette noch die Triebstange oder die Glocke verändert werden. Für einen Wechsel des Nadelhalters liegen daher immer dieselben Verhältnisse an der Manschette, der Triebstange und der Glocke vor, wodurch die Handhabung des Hautpunktierungsgerätes bei Wartungsarbeiten, beispielsweise dem Austausch des Nadelhalters, vereinfacht wird.

Das seitliche Öffnen der Manschette kann grundsätzlich über verschiedene Ausführungen erfolgen. Beispielsweise kann ein Teil der Manschette von dieser entfernbar ausgebildet sein. In dieser einfachsten Ausführungsform wird der bewegliche Anteil der Manschette von dieser abmontiert und gibt die seitlich angeordnete, quer zur Punktionsrichtung ausgerichtete Öffnung der Manschette frei. Der bewegliche Anteil der Manschette kann beispielsweise mittels herkömmlicher Befestigungsmittel oder einer oder mehrerer Rastverbindungen an der Manschette befestigt sein. Alternativ ist es ebenfalls möglich, den beweglichen Teil (Deckel) der Manschette schwenkbar am Hauptteil (Halteteil) zu lagern, beispielsweise mittels eines Scharniers. In dieser Ausführungsform wird der bewegliche Teil der Manschette zur Seite weggeklappt und gibt so die Öffnung der Manschette frei. In einer bevorzugten Ausführungsform ist der Halteteil der Manschette fest mit dem Anschlussstück und über dieses mit dem Antriebsmodul des Hautpunktierungsgerätes verbunden. Der Halteteil kann beispielsweise einstückig mit dem Anschlussstück ausgebildet sein. Der Deckel der Manschette ist gegenüber dem Halteteil beweglich gelagert und an diesem mittels wenigstens eines Scharniers befestigt. Dies hat den Vorteil, dass auch bei geöffneter Manschette sämtliche Bestandteile der Manschette am Nadelmodul verbleiben und keine losen Teile verloren gehen können.

Die Manschette weist zweckmäßig wenigstens einen Verschluss auf, der sie in der die Triebstange und die Glocke umschließenden Position hält und verhindert dass sich die von der Manschette gehaltenen Teile des Nadelmoduls unbeabsichtigt lösen. Die Ausgestaltung des Verschlusses der Manschette ist nicht besonders beschränkt. Bevorzugt sind Verschlüsse, die sich vom Bediener schnell und unkompliziert lösen und wieder schließen lassen. Da die Öffnung der Manschette quer zur Punktionsrichtung erfolgt, wird der Verschluss durch die Auf- und Abbewegung in Punktionsrichtung praktisch nicht mit Kräften beaufschlagt und muss daher nicht besonders stark ausgebildet sein. So kann beispielsweise wenigstens ein magnetischer Verschluss verwendet werden, der besonders einfach zu öffnen und zu schließen ist.

Die Manschette kann zur Aufnahme der gesamten Glocke ausgebildet sein. Es reicht jedoch aus, wenn nur ein Teil der Glocke von der Manschette umschlossen und von dieser festgehalten wird. So kann der Bediener die Glocke festhalten, während er mit der anderen Hand die Manschette löst. Die Glocke muss mindestens einen Abschnitt aufweisen, der groß genug ist, um den Nadelhalter aufzunehmen. Um diesen Abschnitt zu umschließen, müsste die Manschette vergleichsweise groß ausgebildet sein. Um eine kompakte Ausführungsform und gleichzeitig eine optisch ansprechende Gestaltung des Hautpunktierungsgerätes zu erreichen, ist es im Gegensatz dazu daher bevorzugt, dass die Glocke einen Glockenkörper und einen gegenüber dem Glockenkörper verjüngten Glockenhals aufweist, wobei die Manschette ausschließlich den Glockenhals umschließt. Der Glockenkörper ist dabei derjenige Bereich der Glocke, der zur Aufnahme des Nadelhalters ausgebildet ist. Der Glockenhals dagegen ist lediglich dazu ausgebildet, die Triebstange aufzunehmen und darin gleitend zu lagern. Dadurch kann der Glockenhals deutlich schmaler bzw. kleiner als der Glockenkörper ausgeführt werden.

Zur Befestigung der Glocke an der Manschette weisen beide Teile bevorzugt komplementäre Befestigungsmittel auf. Komplementär bedeutet, dass die Befestigungsmittel formschlüssig ineinander eingreifen und so zu einer Befestigung führen, bei der die Glocke in Punktionsrichtung und zweckmäßig auch verdrehsicher in einer definierten Position bezüglich des Antriebsmoduls gehalten wird. Die Haltemittel und die Glockenhalterung umfassen beispielsweise komplementär ausgebildete Vorsprünge bzw. Rücksprünge, die bei der an der Manschette befestigten Glocke formschlüssig ineinandergreifen, insbesondere derart, dass sich die Glocke in Punktionsrichtung nicht mehr bezüglich des Antriebsmoduls verschiebt. Die Haltemittel und die Glockenhalterung verhindern also, dass die Glocke in Punktionsrichtung aus der Manschette herausrutscht bzw. herausgezogen wird. Eine Feststellung der Glocke an der Manschette quer zur Punktionsrichtung wird bereits durch die die Glocke umschließende Manschette, und insbesondere deren Verschluss, bewirkt. Insgesamt lässt sich durch diese Ausführungsform eine verlässliche Befestigung der Glocke an der Manschette erreichen, die insbesondere den im Betrieb des Hautpunktierungsgerätes auftretenden Belastungen in Punktionsrichtung angepasst ist.

Die Triebstange ist derjenige Teil, an der die Nadelhalterung befestigt ist. Die Triebstange überträgt die Auf- und Abbewegung, die von einem Antrieb des Antriebsmoduls kommt, auf den Nadelhalter und die wenigstens eine Nadel. Der Antrieb ist dabei üblicherweise derart ausgebildet, dass er eine Rotationsbewegung in eine lineare Auf- und Abbewegung umwandelt, beispielsweise über wenigstens ein Pleuel. Grundsätzlich könnte die Triebstange also beispielsweise an ein Pleuel des Antriebs des Antriebsmoduls ankuppelbar sein, sodass die Triebstange von diesem Pleuel in die Auf- und Abbewegung in Punktionsrichtung versetzt wird. Da die Triebstange allerdings beim Auswechseln der Glocke und/oder des Nadelhalters mit von der Manschette und dem Nadelmodul entfernbar sein soll, ist es bevorzugt, dass die Triebstange erst räumlich nach der Umwandlung einer Rotationsbewegung in eine linear Auf- und Abbewegung an den Antrieb des Antriebsmoduls ankuppelbar ausgebildet ist. Insbesondere ist bevorzugt, dass die Triebstange eine Antriebskupplung aufweist, mit der die Triebstange an eine Antriebstange des Antriebsmoduls ankuppelbar ist. Mit anderen Worten weist das Antriebsmodul eine Antriebstange auf, die vom Antrieb des Antriebsmoduls in eine lineare Auf- und Abbewegung versetzt wird. Die Umwandlung von einer Rotationsbewegung in eine lineare Auf- und Abbewegung ist also an der Antriebstange des Antriebsmoduls bereits vollzogen. Die Triebstange des Nadelmoduls wird direkt an die Antriebstange des Antriebsmoduls angekuppelt, übernimmt die Auf- und Abbewegung von der Antriebstange und überträgt diese auf den Nadelhalter. Die Antriebskupplung zur Verbindung der Triebstange mit der Antriebstange ist dadurch besonders einfach ausführbar und umfasst im Wesentlichen nur eine Verbindung zwischen der Triebstange und der Antriebstange, die eine Bewegung in Punktionsrichtung übertragen kann. Das Auswechseln der Glocke in Kombination mit der Entkopplung der Triebstange von der Antriebstange und einem nachfolgenden Wiederankuppeln der Triebstange ist in dieser Ausführungsform sehr leicht möglich.

Besonders einfach lässt sich die Triebstange an die Antriebstange an- bzw. von dieser abkuppeln, wenn die Antriebstange mit ihrer Stangenkupplung vom Antriebsmodul des Hautpunktierungsgerätes kommend bis in die Manschette hineinragt. Die Verbindung zwischen Triebstange und Antriebstange befindet sich also in der Manschette, und zwar am besten so , dass die Kupplung der Antriebstange in jeder Phase der Auf- und Abbewegung in der Manschette angeordnet ist. Entsprechend wird die Manschette bevorzugt so dimensioniert, dass sie die Antriebskupplung in jeder Phase der Auf- und Abbewegung umschließt. Die Verbindungsstelle der Triebstange mit der Antriebstange ist also, egal ob sich die wenigstens eine Nadel gerade in der maximal aus der Glocke herausgeschobenen oder in der maximal in die Glocke eingezogenen Position befindet, immer in der Manschette angeordnet. Verfügt das Anschlussstück über eine Einstichtiefenverstelleinrichtung, muss dies bei der Ausgestaltung der Manschette entsprechend berücksichtigt werden. Dadurch, dass die Antriebskupplung und die Stangenkupplung und damit die Verbindungsstelle der Triebstange mit der Antriebstange immer in der Manschette liegt, kann die Kupplung der beiden Stangen bei geöffneter Manschette in jeder Phase der Auf- und Abbewegung schnell und einfach gelöst bzw. wieder verbunden werden. Gleichzeitig wird die Verbindung der Triebstange mit der Antriebstange quer zur Punktionsrichtung von der Manschette gehalten. Es ist daher ausreichend, wenn die Kupplung zwischen der Triebstange und der Antriebstange in Punktionsrichtung gerichtete Zug- und Druckkräfte zwischen den beiden Stangen überträgt. Die Antriebskupplung und die dazu komplementär ausgebildete Stangenkupplung können daher besonders einfach ausgeführt sein.

Die Zeit, die für einen Nadelwechsel benötigt wird, bestimmt sich über die Art, wie der Nadelhalter an der Triebstange befestigt ist. Theoretisch wäre es möglich, den Nadelhalter fest an der Triebstange zu befestigen, beispielsweise die Triebstange einstückig mit dem Nadelhalter auszubilden. Da die Nadeln allerdings nicht nur aus hygienischen Gründen von Zeit zu Zeit zur Reinigung gewechselt werden müssen, sondern auch Verschleiß unterliegen und komplett ersetzt werden müssen, ist es bevorzugt, wenn der Nadelhalter lösbar an der Triebstange befestigt ist, sodass der Nadelhalter getrennt von der Triebstange ausgewechselt und/oder entsorgt werden kann. Die lösbare Verbindung des Nadelhalters an der Triebstange kann dabei auf verschiedene Arten realisiert sein. Beispielsweise könnte der Nadelhalter auf ein Gewinde der Triebstange aufschraubbar sein. Noch einfacher und damit bevorzugt ist es allerdings, wenn der Nadelhalter über eine Rastverbindung an der Triebstange befestigt ist. Die Rastverbindung ist insbesondere derart ausgebildet, dass der Nadelhalter einfach auf die Triebstange aufsteckbar und wieder von dieser abziehbar ist. Gleichzeitig ist die Rastverbindung derart ausgebildet, dass ausgeschlossen ist, dass sich der Nadelhalter im Betrieb des Hautpunktierungsgerätes von alleine löst. Auf diese Weise wird ein besonders zeitsparender Wechsel des Nadelhalters ermöglicht, wobei gleichzeitig bei verschlissenen Nadeln nicht die komplette Triebstange ausgetauscht werden muss.

Je nach Einsatzgebiet des erfindungsgemäßen Hautpunktierungsgerätes können unterschiedliche Arten, Anzahlen und Größen von Nadeln zum Einsatz kommen. In einer Ausführungsform des Nadelmoduls weist der Nadelhalter genau eine Nadel auf. Dabei kann es sich auch um eine Hohlnadel handeln. Gemäß einer weiteren Ausführungsform weist der Nadelhalter mehrere Nadeln auf. Gemäß einer bevorzugten Ausführungsform umfasst das Nadelmodul ein Set aus verschiedenen Nadelhaltern, die jeweils mit unterschiedlichen Nadeln und/oder einer unterschiedlichen Anzahl von Nadeln ausgestattet sind. Im Betrieb des Hautpunktierungsgerätes kommt immer nur einer dieser Nadelhalter des Sets zum Einsatz. Auf diese Weise kann das erfindungsgemäße Nadelmodul in sämtlichen Einsatzgebieten von Hautpunktierungsgeräten Anwendung finden.

Wie schon angesprochen, ist es für die Anwendung des erfindungsgemäßen Hautpunktierungsgerätes wichtig, dass die Einstiche der wenigstens einen Nadel in die Haut möglichst präzise erfolgen und die gewünschten Vorgaben eingehalten werden. Gerade bei der Verwendung von mehr als nur einer Nadel an dem erfindungsgemäßen Nadelmodel ist es daher von Vorteil, wenn das Nadelmodul innerhalb der Glocke nicht um eine Achse parallel zur Punktionsrichtung rotieren kann. Wie schon beschrieben, kann die Verdrehsicherheit einerseits durch die Ausgestaltung der Haltevorrichtung erreicht werden, mit der Glocke und Manschette aneinander befestigt werden. Zusätzlich kann der Nadelhalter einen Körper aufweisen, der derart formschlüssig in dem Innenraum der Glocke aufgenommen ist, dass er sich gegenüber der Glocke nicht dreht. Beispielsweise weist der Nadelhalter eine eckige, insbesondere viereckige, Kontur auf, die in einem Innenraum der Glocke mit entsprechend eckigem, insbesondere viereckigem, Querschnitt aufgenommen wird. Alternativ zur formschlüssigen Lagerung in der Glocke kann eine Rotation der Triebstange - zum Beispiel ebenfalls durch rotationsfreie Lagerung - unterbunden werden und der Nadelhalter verdrehsicher an der Triebstange angebracht sein.

Die Lösung der eingangs genannten Aufgabe gelingt ebenfalls mit einem Hautpunktierungsgerät, welches das vorstehend beschriebene erfindungsgemäße Nadelmodul umfasst und damit die in diesem Zusammenhang bereits beschriebenen Vorteile ebenfalls aufweist.

Nachstehend wird die Erfindung anhand der in den Figuren gezeigten Ausführungsbeispiele näher erläutert. Die Zeichnungen beschreiben lediglich bevorzugte Varianten der Erfindung, ohne auf diese beschränkt zu sein. Gleiche Bezugszeichen bezeichnen gleiche Teile, wobei sich wiederholende Bauteile nicht in jeder Figur gesondert bezeichnet sind. Es zeigen schematisch:
- Fig. 1: eine perspektivische Ansicht eines Hautpunktierungsgerätes;
- Fig. 2: eine Seitenansicht eines Hautpunktierungsgerätes;
- Fig. 3: eine perspektivische Ansicht einer Triebstange;
- Fig. 4: eine perspektivische Ansicht eines Nadelhalters;
- Fig. 5: einen Längsschnitt durch einen Nadelhalter;
- Fig. 6: eine perspektivische Ansicht eines Nadelhalters und einer Triebstange in einer Glocke;
- Fig. 7: einen Längsschnitt durch einen Nadelhalter und eine Triebstange in einer Glocke;
- Fig. 8: eine Detailansicht einer Kupplung zwischen einer Triebstange und einer Antriebstange;
- Fig. 8a-c: verschiedene Detailansichten der Kupplung aus Fig. 8 mit einer Sicherungshülse;
- Fig. 9: eine perspektivische Ansicht eines Deckels einer Manschette;
- Fig. 10: eine perspektivische Ansicht eines Halteteils einer Manschette;
- Fig. 11: eine perspektivische Ansicht eines Halteteils einer Manschette;
- Fig. 12: eine perspektivische Ansicht eines Deckels einer Manschette;
- Fig. 13: eine perspektivische Ansicht einer an dem Halteteil einer Manschette gelagerten Antriebstange, Triebstange und Glocke; und
- Fig. 14: eine perspektivische Ansicht auf eine geschlossene Manschette.

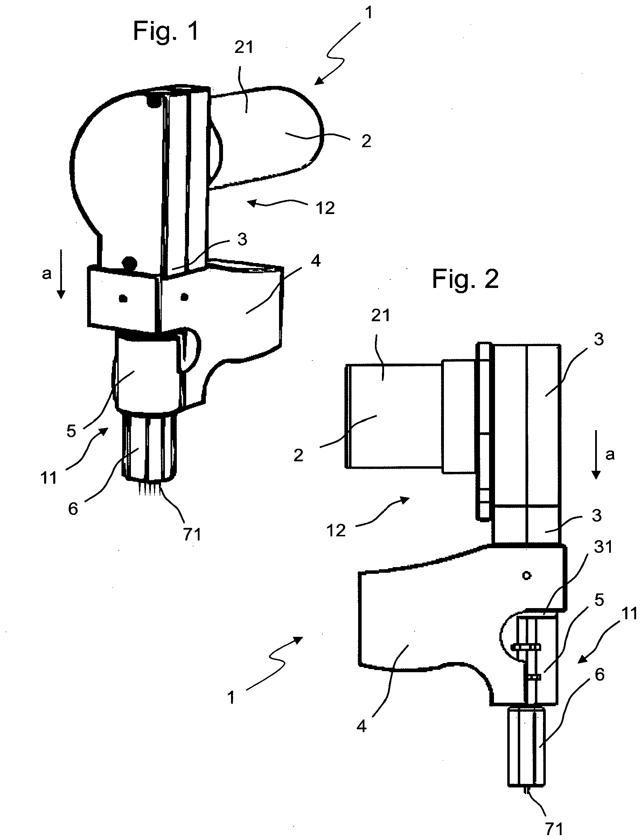

Die Figuren 1 und 2 zeigen ein erfindungsgemäßes Hautpunktierungsgerät 1 zur Behandlung der Haut. Das Hautpunktierungsgerät 1 umfasst ein Nadelmodul 11 und ein Antriebsmodul 12. Das Antriebsmodul 12 wiederum umfasst einen Antrieb 2, beispielsweise einen Elektroantrieb, in einem Antriebsgehäuse 21. Das Antriebsmodul 12 ist über ein zum Nadelmodul 11 gehörendes Anschlussstück 3 mit dem Nadelmodul 11 verbunden. Das Nadelmodul 11 weist neben dem Anschlussstück 3 eine Manschette 5 und eine Glocke 6 auf. Darüber hinaus umfasst das als Handgerät ausgebildete Hautpunktierungsgerät 1 ebenfalls einen Handgriff 4, an dem es von einem Bediener gehalten werden kann. Aus der Glocke 6 ragen Nadeln 71, die durch eine vom Antrieb 2 bereitgestellte Auf- und Abbewegung in Punktionsrichtung a wiederholt in die Glocke 6 eingezogen und wieder aus dieser heraus geschoben werden können. Im Einsatz des Hautpunktierungsgerätes 1 wird dieses mit dem die Öffnung der Glocke 6 umgebenden Rand auf die Haut aufgesetzt, sodass durch die Bewegung der Nadeln 71 in Punktionsrichtung a ein Einstich der Nadeln 71 in die Haut erfolgt.

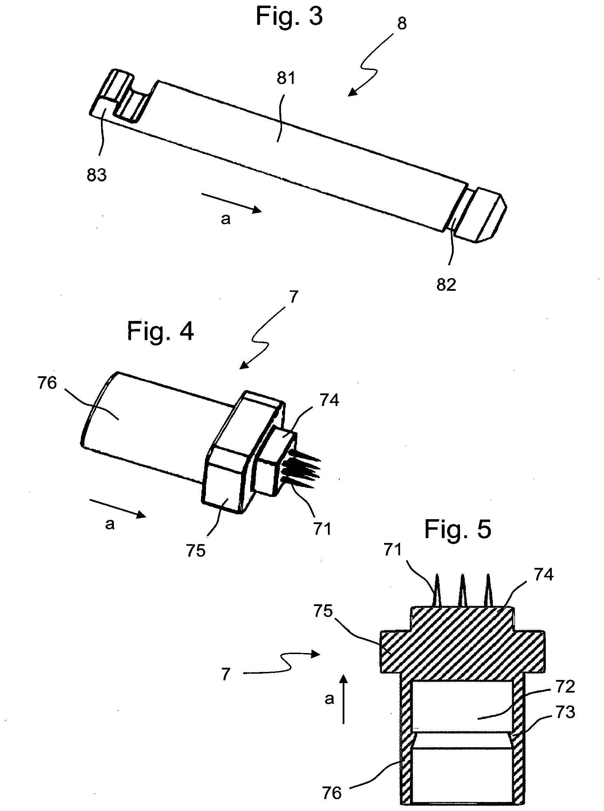

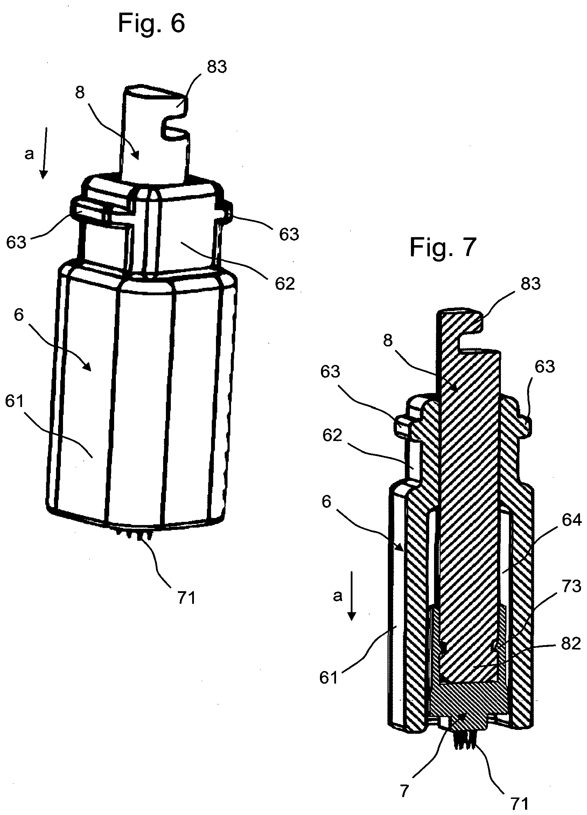

Die Nadeln 71 sind an einem Nadelhalter 7, wie in den Figuren 4 und 5 gezeigt, befestigt. Am Nadelhalter 7 ist ein Stempel 74 angeordnet, an dem sich die Nadeln 71 befinden. Darüber hinaus umfasst der Nadelhalter 7 einen Körper 75 und einen Verbindungsteil 76. Wie in Fig. 5 gezeigt, ist der Verbindungsteil 76 zumindest teilweise hohlzylindrisch ausgebildet und weist in seinem Inneren eine Stangenaufnahme 72 auf, in der ein Ende einer Triebstange 8, wie beispielsweise in Fig. 3 gezeigt, aufnehmbar ist. Die Triebstange 8 weist einen Stangenkörper 81 und eine Rastausnehmung 82 auf, die komplementär zum Rastvorsprung 73 ausgebildet ist, der als umlaufender Ring in der Stangenaufnahme 72 des Verbindungsteils 76 des Nadelhalters 7 ausgebildet ist. Der Nadelhalter 7 kann also einfach auf das Ende der Triebstange 8 aufgesteckt werden und rastet an dieser ein. Insbesondere rastet der Nadelhalter 7 mit seinem Rastvorsprung 73 in die Rastausnehmung 82 der Triebstange 8 ein. Der an der Triebstange 8 eingerastete Nadelhalter 7 wird dann durch die Glocke 6 gesteckt bzw. von dieser aufgenommen, wie in den Figuren 6 und 7 dargestellt. Die Glocke 6 weist einen Glockenkörper 61 und einen gegenüber dem Glockenkörper 61 verjüngten Glockenhals 62 auf. Im Glockenkörper 61 umschließt die Glocke 6 einen Innenraum 64 (siehe Figur 7), in dem der Nadelhalter 7 aufgenommen ist. Aufgrund der im Wesentlichen quaderförmigen Ausgestaltung des Körpers 75, der passgenau im Glockeninnenraum 64 aufgenommen wird, wird ein Verdrehen des Nadelhalters 7 verhindert. Der Nadelhalter 7 ist im Innenraum 64 der Glocke 6 gleitend gelagert und kann in und entgegen der Punktionsrichtung a innerhalb des Innenraums 64 zusammen mit der Triebstange 8 auf und ab bewegt werden. Die Triebstange 8 durchdringt den Glockenhals 62 und ist ebenfalls in diesem in Punktionsrichtung a gleitend gelagert. Die Triebstange 8 überträgt eine vom Antrieb 2 kommende Auf- und Abbewegung in Punktionsrichtung a auf den Nadelhalter 7 und die Nadeln 71.

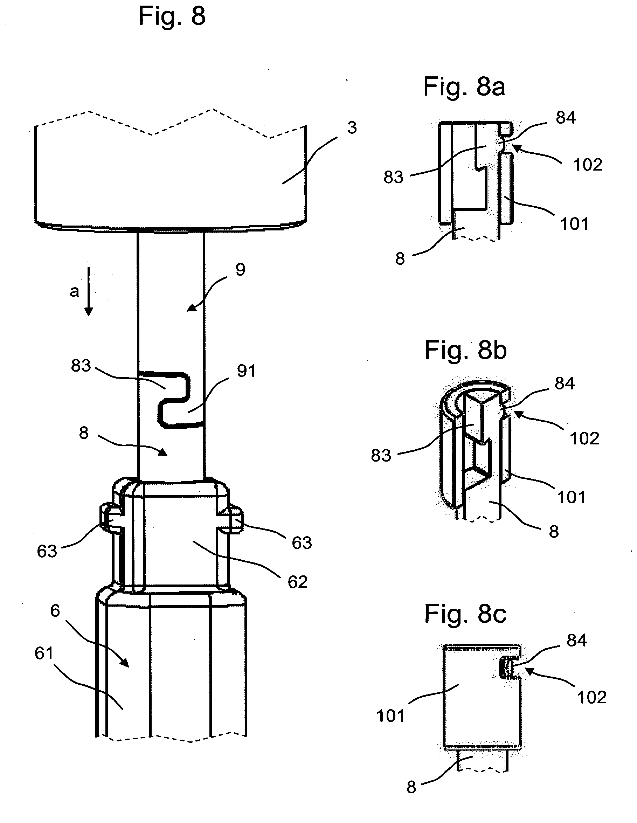

Dafür weist die Triebstange 8 eine Antriebskupplung 83 auf, wie sie im gezeigten Ausführungsbeispiel als Kombination von Vorsprung und Ausnehmung in der Triebstange 8 ausgebildet ist. Wie beispielsweise in Fig. 8 dargestellt, greift die Triebstange 8 mit der Antriebskupplung 83 in eine komplementär ausgebildete Stangenkupplung 91 einer Antriebstange 9 des Antriebs 2 ein. Auch die Stangenkupplung 91 der Antriebstange 9 ist als Kombination von Ausnehmung und Vorsprung ausgebildet, sodass die Antriebskupplung 83 der Triebstange 8 und die Stangenkupplung 91 der Antriebstange 9 formschlüssig ineinandergreifen und dabei eine Verbindung bilden, die Zug- und Druckkräfte in und/oder entgegen der Punktionsrichtung a zwischen der Triebstange 8 und der Antriebstange 9 übertragen kann. Wie ebenfalls in Figur 8 dargestellt, kuppeln die Antriebskupplung 83 und die Stangenkupplung 91 derart miteinander, dass insgesamt eine Außenkontur der Kuppelstelle entsteht, die im Wesentlichen nicht von der Außenkontur des Stangenkörpers 81 der Triebstange 8 abweicht. Die Kuppelstelle kann daher genau wie die Antriebsstange 9 und die Triebstange 8 in der Manschette 5 gleiten, wie nachstehend noch erläutert wird. Die Antriebstange 9 wird vom Antrieb 2 mit einer Auf- und Abbewegung in und entgegen der Punktionsrichtung a beaufschlagt. Diese Auf- und Abbewegung wird von der Antriebstange 9 über die Stangenkupplung 91 und die Antriebskupplung 83 der Triebstange 8 auf die Triebstange 8 übertragen, die die Bewegung schließlich auf den Nadelhalter 7 und damit die Nadeln 71 überträgt. Die in Fig. 8 nicht dargestellte Manschette 5 sichert die Kupplung zwischen der Triebstange 8 und der Antriebstange 9 gegen unbeabsichtigtes Lösen, wie nachfolgend erläutert werden wird.

Die Figuren 8a, 8b und 8c zeigen eine optional vorhandene Sicherungshülse 101, die die Kuppelstelle zwischen der Antriebskupplung 83 der Triebstange 8 und der Stangenkupplung 91 der Antriebsstange 9 zumindest teilweise und insbesondere vollständig umschließt. Auf eine Darstellung der Stangenkupplung 91 und der Triebstange 9 wurde in den Figuren 8a bis 8c aus Übersichtlichkeitsgründen verzichtet. Die Sicherungshülse 101 ist zweckmäßig aus einem beispielsweise elastischem Kunststoff hergestellt und kann entlang der Triebstange 8 beziehungsweise der Antriebsstange 9 verschoben werden. Beispielsweise wird die Sicherungshülse 101 auf die Triebstange 8 aufgesteckt. Sodann wird die Antriebskupplung 83 in die Stangenkupplung 91 eingehängt, sodass eine Kupplung zwischen Triebstange 8 und Antriebsstange 9 entsteht. Die Sicherungshülse 101 kann dann über die Kuppelstelle geschoben werden, so dass sie die Antriebskupplung 83 und die Stangenkupplung 91 aneinander fixiert. Somit verhindert die Sicherungshülse 101, dass die Antriebskupplung 83 sich versehentlich von der Stangenkupplung 91 löst. Darüber hinaus weisen die Triebstange 8 und die Sicherungshülse 101 komplementäre Befestigungselemente auf, um die Sicherungshülse 101 an der Kuppelstelle zu fixieren. Im gezeigten Ausführungsbeispiel weist die Triebstange 8 einen Vorsprung 84 auf, der in eine komplementäre Ausnehmung 102 in der Sicherungshülse 101 eingreift, wenn die Sicherungshülse 101 die Kuppelstelle umgibt. Die Sicherungshülse 101 ist dabei derart elastisch ausgebildet, dass sie von der Triebstange 8 kommend über den Vorsprung 84 geschoben werden kann, bis dieser in die Ausnehmung 102 eingreift und die Sicherungshülse 101 in dieser Position fixiert. Ist die Verwendung einer Sicherungshülse 101 vorgesehen, so ist die Manschette 5 mit einem Freiraum ausgebildet, in dem sich die Sicherungshülse 101 im Betrieb des Hautpunktierungsgerätes 1 mit der Kupplungsstelle zwischen Antriebsstange 9 und Triebstange 8 auf und ab bewegen kann. Die Manschette 5 ist in diesem Fall mit anderen Worten mindestens um die Dicke der Sicherungshülse 101 von der Triebstange 8 und der Antriebsstange 9 beabstandet ausgebildet.

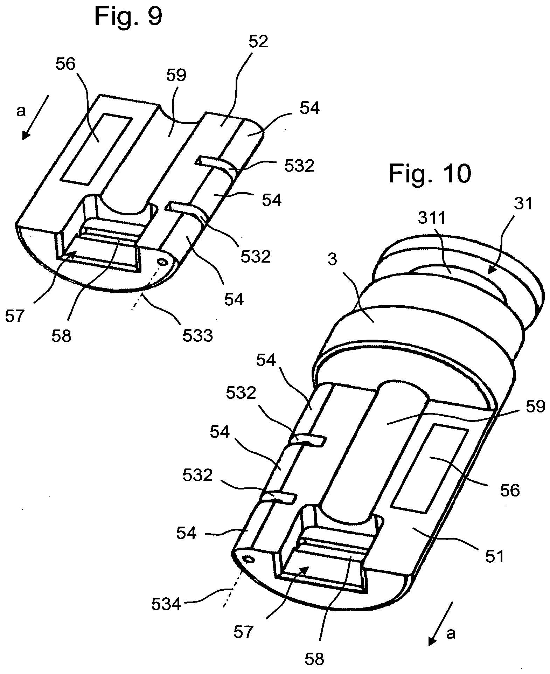

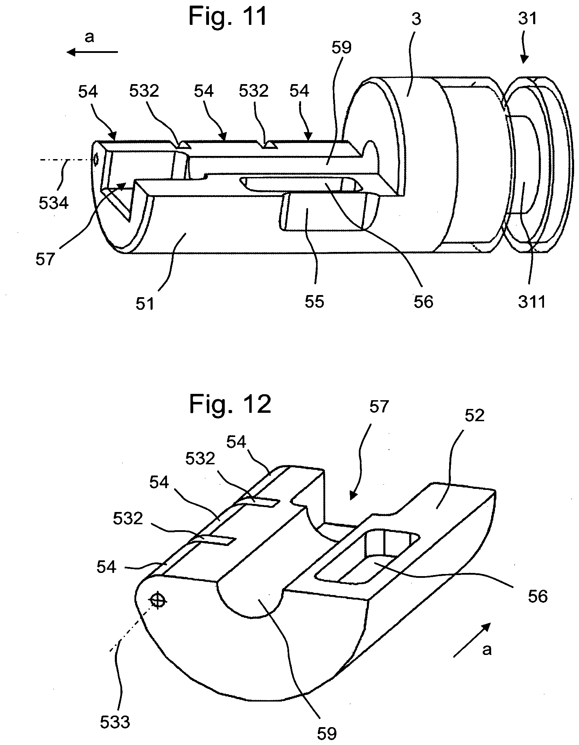

Die Manschette 5, insbesondere deren Halteteil 51 und der Deckel 52, ist in den Figuren 9, 10, 11 und 12 dargestellt. Der Halteteil 51 (Figuren 10, 11) ist mit dem Anschlussstück 3 verbunden und insbesondere einstückig mit diesem ausgebildet. Das Anschlussstück 3 ist in seiner Länge in Punktionsrichtung a verstellbar ausgebildet. Dafür weist das Anschlussstück 3 eine Einstichtiefenverstelleinrichtung 31 auf, die insbesondere in der gezeigten Ausführungsform ein Gewinde 311 umfasst. Durch ein Verstellen des Gewindes 311 wird die Länge des Anschlussstücks 3 in Punktionsrichtung a entweder verkürzt oder verlängert. Da sich die Länge der Antriebstange 9 und der Triebstange 8 nicht ändern, kann durch eine derartige Verstellung der Einstichtiefenverstelleinrichtung 31 die Einstichtiefe der Nadeln 71 verändert werden. Mit anderen Worten wird eingestellt, wie weit die Nadeln 71 in ihrer maximal ausgefahrenen Position während der Auf- und Abbewegung durch den Antrieb 2 aus der Glocke 6 herausragen und über den Öffnungsrand vorstehen. Je nach Anwendung des Hautpunktierungsgerätes 1 kann so die benötigte Einstichtiefe gewählt werden. Das Halteteil 51 weist eine Stangenaufnahme 59 auf, in der die Triebstange 8, die Antriebstange 9 sowie die Stangenkupplung 91 und die Antriebskupplung 83 aufnehmbar sind. Darüber hinaus weist der Halteteil 51 eine Glockenaufnahme 57 auf, in die der Glockenhals 62 aufnehmbar ist. Wie aus den Figuren 6, 7 und 8 hervorgeht, weist der Glockenhals 62 Haltemittel 63 auf, die im gezeigten Ausführungsbeispiel als sich am Glockenhals 62 gegenüberliegende Vorsprünge ausgebildet sind. Diese Haltemittel 63 sind komplementär ausgebildet zu einer an der Glockenaufnahme 57 ausgebildeten Glockenhalterung 58, die im gezeigten Ausführungsbeispiel als Ausnehmung ausgestaltet ist. Insgesamt ist die Glockenaufnahme 57 also als Ausnehmung in der Manschette 5 ausgebildet, die der Außenkontur des Glockenhalses 62 angepasst ist. Die Anordnung verhindert ein Verdrehen der Glocke in ihrer Umfangsrichtung relativ zum Anschlussstück 3.

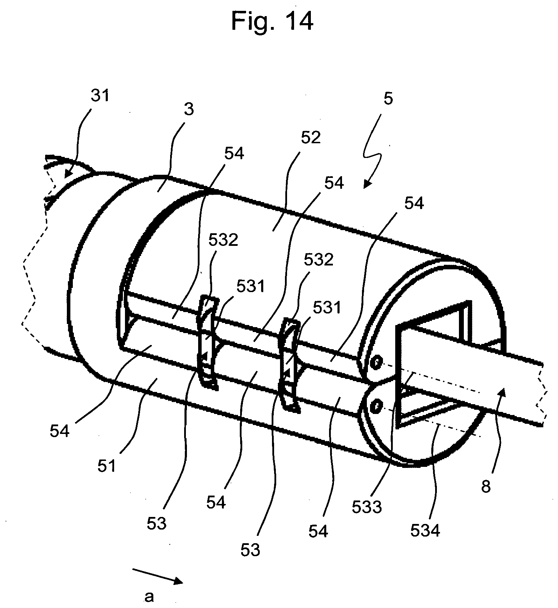

Der Halteteil 51 der Manschette ist über ein Scharnier 53 (Fig. 14) mit dem Deckel 52 (Figuren 9 und 12) verbunden. Dafür weisen sowohl der Halteteil 51 als auch der Deckel 52 Scharnieraufnahmen 532 auf. Diese dienen der gelenkigen Verbindung des Deckels 52 mit dem Halteteil 51, wie nachstehend noch näher erläutert. Die Manschette 5 weist einen Verschluss 56 auf, der im gezeigten Ausführungsbeispiel als magnetischer Verschluss 56 am Halteteil 51 und dem Deckel 52 ausgebildet ist. In den Figuren 11 und 12 ist lediglich die Ausnehmung zu sehen, in die der Magnet des Halteteils 51 und des Deckels 52 jeweils eingefügt wird. Fig. 11 zeigt darüber hinaus eine Griffhilfe 55 am Halteteil 51, die als Ausnehmung auf der den Scharnieraufnahmen 532 gegenüberliegenden Seite des Halteteils 51 ausgebildet ist. Diese Griffhilfe 55 ermöglicht es einem Benutzer, bei auf dem Halteteil 51 befestigten Deckel 52 unter den Rand des Deckels 52 zu greifen und damit auf sehr einfache Weise den Verschluss 56 zu lösen und den Deckel 52 vom Halteteil 51 weg zu schwenken, sodass die Manschette 5 quer zur Punktionsrichtung a geöffnet wird. Der Deckel 52 weist ebenfalls eine Stangenaufnahme 59 und eine Glockenaufnahme 57 auf, die symmetrisch und komplementär zur Stangenaufnahme 59 und Glockenaufnahme 57 des Halteteils 51 ausgebildet sind.

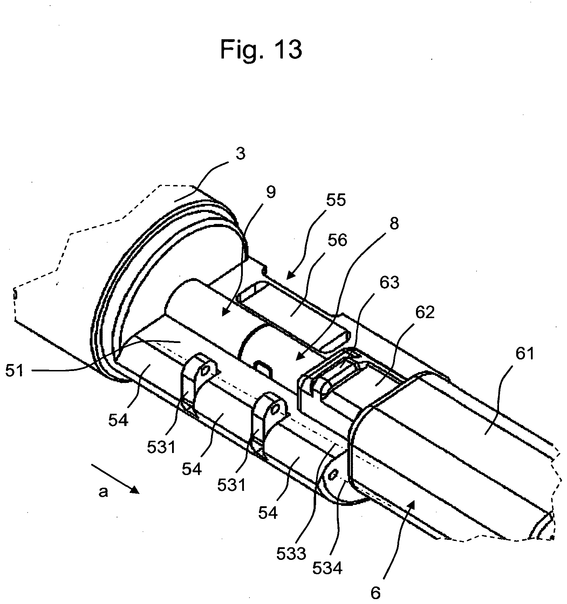

Fig. 13 zeigt eine geöffnete Manschette 5, deren Deckel 52 aus Übersichtlichkeitsgründen nicht dargestellt ist. Die Antriebstange 9, die Triebstange 8 und die Glocke 6 sind in Fig. 13 derart in die Manschette 5 bzw. den Halteteil 51 der Manschette 5 eingelegt dargestellt, wie sie bei geschlossener Manschette 5 von dieser umschlossen sind. Insbesondere ist die Triebstange 8 an die Antriebstange 9 angekuppelt, wie in Fig. 8 dargestellt. Sowohl die Antriebskupplung 83 als auch die Stangenkupplung 91 befinden sich in der Stangenaufnahme 59 der Manschette 5. Der Glockenhals 62 der Glocke 6 ist in die Glockenaufnahme 57 der Manschette 5 eingelegt. Die Haltemittel 63 der Glocke 6 greifen in die Glockenhalterung 58 der Manschette 5, insbesondere des Halteteils 51, ein. Durch diesen Formschluss wird insbesondere eine Bewegung der Glocke 6 in Punktionsrichtung a verhindert. Eine Bewegung der Glocke 6 und der Triebstange quer zur Punktionsrichtung a wird dagegen durch den Deckel 52 der Manschette 5 in der geschlossenen Position verhindert.

Dies geht insbesondere aus Fig. 14 hervor, in der die Glocke 6 aus Übersichtlichkeitsgründen nicht dargestellt ist. Der Deckel 52 wird über die in die Scharnieraufnahmen 532 aufgenommenen Zweilochplatten 531 mit dem Halteteil 51 verbunden. Durch die Verbindung des Halteteils 51 mit dem Deckel 52 über die Zweilochplatten 531 entsteht ein Scharnier 53 mit einer ersten Schwenkachse 533, die durch den Deckel 52 verläuft, und einer zweiten Schwenkachse 534, die durch das Halteteil 51 verläuft. Die Schwenkachsen 533, 534 sind parallel zueinander. Der Deckel 52 kann also über die Scharniere 53 sowohl um die erste Schwenkachse 533 als auch um die zweite Schwenkachse 534 geschwenkt werden. Um diese Schwenkbewegung zu erleichtern und zu unterstützen, ist auf der Seite des Scharniers 53 sowohl am Deckel 52 als auch am Halteteil 51 eine Rundung 54 vorgesehen. Hier sind die beiden aneinander liegenden Kanten auf der Scharnierseite des Deckels 52 und des Halteteils 51 derart abgerundet, dass kein Verklemmen des Deckels 52 gegenüber dem Halteteil 51 möglich ist.

Aus Fig. 14 geht hervor, dass die Außenkontur der Manschette 5 im geschlossenen Zustand, sprich in der die Antriebstange 8 und die Glocke 6 umschließenden Positionen der Manschette 5, im Wesentlichen bündig mit dem Anschlussstück 3 abschließt. Die Manschette 5 weist also eine zylinderförmige Außenkontur auf, die dem Anschlussstück 3 angepasst ist. In dieser Position fixiert die Manschette 5 die Triebstange 8 und die Glocke 6 am Nadelmodul 11, und zwar insbesondere in einer Richtung quer zur Punktionsrichtung a. In Punktionsrichtung a sind die Triebstange 8 formschlüssig über die Antriebskupplung 83 mit der Antriebstange 9 sowie die Glocke 6 über die Haltemittel 63 und die Glockenhalterung 58 mit der Manschette 5 verbunden. Insgesamt sind somit die Triebstange 8 und die Glocke 6 durch die Manschette 5 am Nadelmodul 11 des Hautpunktierungsgerätes 1 befestigt. Wenn die Manschette 5 dagegen an einer Seite quer zur Punktionsrichtung a geöffnet ist (s. Fig. 13), können die Triebstange 8 und die Glocke 6 von der Manschette entfernt werden. Insbesondere können die Triebstange 8 und die Glocke 6 einfach durch eine Bewegung quer zur Punktionsrichtung a aus der Manschette 5 genommen werden. Auf die beschriebene Weise ist das Herausnehmen von Triebstange 8 und Glocke 6 zusammen mit dem Nadelhalter 7 besonders leicht möglich.

## Patentansprüche

1. Nadelmodul (11) zum Anschluss an ein Antriebsmodul (12) eines Hautpunktierungsgerätes (1), mit
- einem am Antriebsmodul (12) befestigbaren Anschlussstück (3),
- einem an einer Triebstange (8) befestigten Nadelhalter (7) mit wenigstens einer Nadel (71), wobei die Triebstange (8) derart mit einem Antrieb (2) des Antriebsmoduls (12) verbindbar ausgebildet ist, dass sie eine vom Antrieb (2) kommende Auf- und Abbewegung auf den Nadelhalter (7) überträgt,
- einer den Nadelhalter (7) und einen Teil der Triebstange (8) aufnehmenden Glocke (6), die derart ausgebildet ist, dass die wenigstens eine Nadel (71) des Nadelhalters (7) an einer geöffneten Seite durch die Auf- und Abbewegung in Punktionsrichtung (a) aus einem Innenraum (64) der Glocke (6) herausgeschoben und wieder in diesen eingezogen werden kann, und
- einer die Triebstange (8) und die Glocke (6) umschließenden, am Anschlussstück (3) befestigten Manschette (5),
wobei die Triebstange (8) in Punktionsrichtung (a) gleitend in der Glocke (6) und in der Manschette (5) gelagert ist, wobei die Manschette (5) an einer Seite quer zur Punktionsrichtung (a) öffenbar ist, so dass die Triebstange (8) und die Glocke (6) aus der Manschette (5) entfernbar sind.

2. Nadelmodul (11) nach Anspruch 1,
**dadurch gekennzeichnet,**
**dass** die Länge des Anschlussstückes (3) in Punktionsrichtung (a) über eine Einstichtiefenverstelleinrichtung (31), die insbesondere als Gewinde (321) ausgebildet ist, verstellbar ist.

3. Nadelmodul (11) nach einem der vorhergehenden Ansprüche,
**dadurch gekennzeichnet,**
**dass** die Manschette (5) zweiteilig ausgebildet ist, derart, dass sie einen Halteteil (51) und einen über ein Scharnier (53) mit dem Halteteil (51) verbundenen und gegenüber diesem beweglich gelagerten Deckel (52) umfasst.

4. Nadelmodul (11) nach einem der vorhergehenden Ansprüche,
**dadurch gekennzeichnet,**
**dass** die Manschette (5) wenigstens einen magnetischen Verschluss (56) aufweist, der die Manschette (5) in der die Triebstange (8) und die Glocke (6) umschließenden Position hält.

5. Nadelmodul (11) nach einem der vorhergehenden Ansprüche,
**dadurch gekennzeichnet,**
**dass** die Glocke (6) einen Glockenkörper (61) und einen gegenüber dem Glockenkörper (61) verjüngten Glockenhals (62) aufweist, wobei die Manschette (5) ausschließlich den Glockenhals (62) umschließt.

6. Nadelmodul (11) nach einem der vorhergehenden Ansprüche,
**dadurch gekennzeichnet,**
**dass** die Glocke (6), insbesondere am Glockenhals (62) ausgebildete, Haltemittel (63) und die Manschette (5) eine Glockenhalterung (58) umfasst, wobei die Haltemittel (63) und die Glockenhalterung (58) derart komplementär zueinander ausgebildet sind, dass sie die Glocke (6) in der die Triebstange (8) und die Glocke (6) umschließenden Position der Manschette (5) in Punktionsrichtung (a) feststellen.

7. Nadelmodul (11) nach einem der vorhergehenden Ansprüche,
**dadurch gekennzeichnet,**
**dass** die Triebstange (8) eine Antriebskupplung (83) aufweist, mit der die Triebstange (8) an eine Antriebsstange (9) des Antriebsmoduls (12) ankuppelbar ist.

8. Nadelmodul (11) nach Anspruch 7,
**dadurch gekennzeichnet,**
**dass** eine insbesondere elastische Sicherungshülse (101) vorhanden ist, welche über die Antriebskupplung (83) geschoben werden kann.

9. Nadelmodul (11) nach Anspruch 7 oder 8,
**dadurch gekennzeichnet,**
**dass** die Manschette (5) ausgebildet ist, die Antriebskupplung (83) und gegebenenfalls die Sicherungshülse (101) in jeder Phase der Auf- und Abbewegung zu umschließen.

10. Nadelmodul (11) nach einem der vorhergehenden Ansprüche,
**dadurch gekennzeichnet,**
**dass** der Nadelhalter (7) über eine Rastverbindung an der Triebstange (8) befestigt ist.

11. Nadelmodul (11) nach einem der vorhergehenden Ansprüche,
**dadurch gekennzeichnet,**
**dass** der Nadelhalter (7) mehrere Nadeln (71) aufweist.

12. Nadelmodul (11) nach einem der vorhergehenden Ansprüche,
**dadurch gekennzeichnet,**
**dass** der Nadelhalter (7) einen Körper (75) aufweist, der derart formschlüssig in einem Innenraum (64) der Glocke (6) aufgenommen ist, dass die Lagerung des Nadelhalters (7) in der Glocke (6) rotationsfrei ist.

13. Hautpunktierungsgerät (1) mit einem Nadelmodul (11) nach einem der vorhergehenden Ansprüche.

## Claims

1. A needle module (11) for connection to a drive module (12) of a skin puncturing apparatus (1), with:
- a connector (3) attachable to the drive module (12),
- a needle holder (7) attached to a connecting rod (8) and having at least one needle (71), said connecting rod (8) being configured so as to be connectable to a drive (2) of the drive module (12) in such a manner that it transfers an up-and-down movement coming from the drive (2) to the needle holder (7),
- a cap (6) accommodating the needle holder (7) and a portion of the connecting rod (8), said cap being configured in such a manner that the at least one needle (71) of the needle holder (7) can be extended out of and retracted into an interior (64) of the cap (6) on an open side by means of the up-and-down movement in a puncturing direction (a), and
- a collar (5) attached to the connector (3) and enclosing the connecting rod (8) and the cap (6),
wherein the connecting rod (8) is mounted inside the cap (6) and the collar (5) so as to be glidable in the puncturing direction (a),
wherein the collar (5) can be opened on a side perpendicular to the puncturing direction (a) so that the connecting rod (8) and the cap (6) can be removed from the collar (5).

2. The needle module (11) according to claim 1,
**characterized in that**
the length of the connector (3) in the puncturing direction (a) is adjustable via a puncturing depth adjusting device (31), which is configured in particular as a thread (321).

3. The needle module (11) according to one of the preceding claims,
**characterized in that**
the collar (5) is configured as two pieces in such a way that it comprises a handle portion (51) and a lid (52) which is connected to the handle portion (51) via a hinge (53) and mounted in a movable manner in relation to the handle portion.

4. The needle module (11) according to one of the preceding claims,
**characterized in that**
the collar (5) comprises at least one magnetic fastener (56) that holds the collar (5) in the position in which it encloses the connecting rod (8) and the cap (6).

5. The needle module (11) according to one of the preceding claims,
**characterized in that**
the cap (6) includes a cap body (61) and a cap neck (62) which is tapered in relation to the cap body (61), wherein the collar (5) encloses the cap neck (62) only.

6. The needle module (11) according to one of the preceding claims,
**characterized in that**
the cap (6) comprises holding means (63) formed in particular at the cap neck (62) and the collar (5) comprises a cap socket (58), said holding means (63) and said cap socket (58) being configured in a complementary manner relative to one another so that they lock the cap (6) in the position of the collar (5) in which the latter encloses the connecting rod (8) and the cap (6) in the puncturing direction (a).

7. The needle module (11) according to one of the preceding claims,
**characterized in that**
the connecting rod (8) comprises a drive coupling (83) with which the connecting rod (8) can be coupled to a driving rod (9) of the drive module (12).

8. The needle module (11) according to claim 7,
**characterized in that**
a retaining sleeve (101), in particular an elastic retaining sleeve (101), is provided which can be slid over the drive coupling (83).

9. The needle module (11) according to claim 7 or 8,
**characterized in that**
the collar (5) is configured to enclose the drive coupling (83) and, if applicable, the retaining sleeve (101) in each phase of the up-and-down movement.

10. The needle module (11) according to one of the preceding claims,
**characterized in that**
the needle holder (7) is attached to the connecting rod (8) via a latch connection.

11. The needle module (11) according to one of the preceding claims,
**characterized in that**
the needle holder (7) comprises multiple needles (71).

12. The needle module (11) according to one of the preceding claims,
**characterized in that**
the needle holder (7) comprises a body (75) accommodated in a form-locking manner in an interior (64) of the cap (6) so that the needle holder (7) is mounted inside the cap (6) in such a manner that it cannot be rotated.

13. A skin puncturing apparatus (1) with a needle module (11) according to one of the preceding claims.

## Revendications

1. Module d'aiguille (11) destiné à un raccordement avec un module d'entraînement (12) d'un dispositif (1) pour percer la peau, avec :
- un connecteur (3) pouvant être fixé au module d'entraînement (12),
- un porte-aiguille(s) (7) fixé à une biellette (8) et ayant au moins une aiguille (71), ladite biellette (8) étant configurée de façon à pouvoir être raccordée à un entraînement (2) du module d'entraînement (12) d'une manière telle qu'elle transmette au porte-aiguille(s) (7) un mouvement de va-et-vient venant de l'entraînement (2),
- un capuchon (6) accueillant le porte-aiguilles (7) et une partie de la biellette (8), ledit capuchon étant configuré de telle manière que ladite au moins une aiguille (71) du porte-aiguille(s) (7) puisse être sortie d'un, et rétractée dans un, volume intérieur (64) du capuchon (6) sur un côté ouvert, grâce au mouvement de va-et-vient dans une direction de perforation (a), et
- un manchon (5) fixé au connecteur (3) et entourant la biellette (8) et le capuchon (6),
dans lequel la biellette (8) est montée à l'intérieur du capuchon (6) et du manchon (5) de manière à pouvoir coulisser dans la direction de perforation (a),
dans lequel le manchon (5) peut être ouvert sur un côté perpendiculaire à la direction de perforation (a) de sorte que la biellette (8) et le capuchon (6) puissent être retirés du manchon (5).

2. Module d'aiguille (11) selon la revendication 1,
***caractérisé en ce que***
la longueur du connecteur (3) dans la direction de perforation (a) est réglable via un dispositif (31) de réglage de la profondeur de perforation qui est configuré en particulier sous la forme d'un filetage (321).

3. Module d'aiguille (11) selon l'une quelconque des revendications précédentes,
***caractérisé en ce que***
le manchon (5) est configuré en deux parties d'une façon telle qu'il comprend une partie formant poignée (51) et un couvercle (52) qui est raccordé à la portion formant poignée (51) par une charnière (53) et est monté de manière mobile par rapport à la partie formant poignée.

4. Module d'aiguille (11) selon l'une quelconque des revendications précédentes,
***caractérisé en ce que***
le manchon (5) comprend au moins une attache magnétique (56) qui maintient le manchon (5) dans la position dans laquelle il entoure la biellette (8) et le capuchon (6).

5. Module d'aiguille (11) selon l'une quelconque des revendications précédentes,
***caractérisé en ce que***
le capuchon (6) comprend un corps (61) de capuchon et un col (62) de capuchon qui est effilé en relation avec le corps (61) du capuchon, dans lequel le manchon (5) recouvre uniquement le col (62) du capuchon.

6. Module d'aiguille (11) selon l'une quelconque des revendications précédentes,
***caractérisé en ce que***
le capuchon (6) comprend des moyens de retenue (63) formés en particulier au niveau du col (62) du capuchon et le manchon (5) comprend un support (58) de capuchon, lesdits moyens de retenue (63) et ledit support (58) de capuchon étant configurés de manière complémentaire entre eux de façon à ce qu'ils verrouillent le capuchon (6) dans la position du manchon (5) dans laquelle ce dernier recouvre la biellette (8) et le capuchon (6) dans la direction de perforation (a).

7. Module d'aiguille (11) selon l'une quelconque des revendications précédentes,
***caractérisé en ce que***
la biellette (8) comprend un accouplement (83) avec lequel la biellette (8) peut être accouplée à une bielle de commande (9) du module d'entraînement (12).

8. Module d'aiguille (11) selon la revendication 7,
***caractérisé en ce que***
une douille de retenue (101), en particulier une douille de retenue élastique (101), est prévue, qui peut glisser sur l'accouplement (83).

9. Module d'aiguille (11) selon la revendication 7 ou 8,
***caractérisé en ce que***
le manchon (5) est configuré pour recouvrir l'accouplement (83) et, le cas échéant, la douille de retenue (101) dans chaque phase du mouvement de va-et-vient.

10. Module d'aiguille (11) selon l'une quelconque des revendications précédentes,
***caractérisé en ce que***
le porte-aiguille(s) (7) est fixé à la biellette (8) via une liaison d'encliquetage.

11. Module d'aiguille (11) selon l'une quelconque des revendications précédentes,
***caractérisé en ce que***
le porte-aiguille(s) (7) comprend une pluralité d'aiguilles (71).

12. Module d'aiguille (11) selon l'une quelconque des revendications précédentes,
***caractérisé en ce que***
le porte-aiguille(s) (7) comprend un corps (75) reçu avec complémentarité de formes dans un volume intérieur (64) du capuchon (6), de telle sorte que le porte-aiguille(s) (7) est monté à l'intérieur du capuchon (6) sans pouvoir être entraîné en rotation.

13. Dispositif (1) pour percer la peau, avec un module d'aiguille (11) selon l'une quelconque des revendications précédentes.
